# EUROPEAN PATENT SPECIFICATION

(11) **EP 0 533 222 B1**
(45) Date of publication and mention of the grant of the patent: **04.12.1996**
(21) Application number: 92202214.0
(22) Date of filing: 17.07.1992
(51) Int. Cl.: C07K 5/075, A23L 1/236

(54) **Method for crystallizing alpha-L-aspartyl-L-phenylalanine methyl ester**
Verfahren zur Kristallisation von Alpha-L-Aspartyl-L-Phenylalaninmethylester
Procédé de cristallisation de l'alpha-l-aspartyl-l-phénylalanine ester méthylique

(30) Priority: 19.07.1991 JP 203703/91; 29.07.1991 JP 210469/91; 19.11.1991 JP 329851/91
(43) Date of publication of application: 24.03.1993
(73) Proprietor: HOLLAND SWEETENER COMPANY V.O.F., NL-6212 XW Maastricht (NL)
(72) Inventor: Murakami, Tsuguo, Yamaguchi, 746 (JP); Vermijs, Winfried Johannes Wouterus, 6164 BE Geleen (NL); Egashira, Hidetaka, Yamaguchi, 746 (JP); Okajima, Kengo, Yamaguchi, 746 (JP); Wakamatsu, Hidetoshi, 6245 CJ Eijsden (NL)
(74) Representative: den Hartog, Jeroen Hendrikus Joseph

(56) References cited:
- JOURNAL OF CHEMICAL TECHNOLOGY AND BIOTECHNOLOGY vol. 43, 1988, pages 71 - 82; SHIN'ICHI KISHIMOTO ET AL.: 'A Process Development for the Bundling Crystallization of Aspartame'

## Description

This invention relates to a method for crystallizing α-L-aspartyl-L-phenylalanine methyl ester(hereinafter referred to as "APM") by cooling, which is expected to find a wide application as a low-calorie sweetener because of its sweetness of good quality which is about 200 times as sweet as sugar.

### Prior Art

APM can be synthesized by various methods. Among industrial production processes for producing APM are included the one in which an N-protected aspartic acid is condensed with phenylalanine methyl ester in the presence of an enzyme, followed by the elimination of the protecting group (US-A-4,282,721), and the one in which an N-protected aspartic acid anhydride is condensed with phenylalanine methyl ester in an organic solvent, and the protecting group is eliminated in accordance with a conventional method (US-A-3,786,039).

In any process, a crystallization step is indispensable to produce the final product by isolating APM from the reaction mixture. The crystallization step is ordinarily carried out by (1) precipitating APM crystals through cooling of (a) an APM solution obtained via synthesis and purification steps or (b) an APM solution prepared by redissolving a crude APM product into water, an organic solvent or a water-containing organic solvent, (ii) subjecting the precipitated crystals to solid-liquid separation and dewatering by use, e.g. of a centrifugal separator, and then (iii) drying the dewatered crystals to give the final product.

Such a crystallization through cooling is usually carried out by using (i) a crystallizer provided with a stirrer and a cooling/heat-transfer surface or (ii) a crystallizer provided with a heat-exchanger of external circulation type. There is also known a method in which crystallization is effected through conductive heat transfer without forced flow to improve the crystal properties of APM(EP-B-91.787).

However, when APM is crystallized through cooling in a crystallizer which utilizes forced flow of, e.g. ordinary mechanical stirring or external circulation, crystals are obtained, which are poor in solid-liquid separability or dewatering properties. Such crystals readily adhere to the cooling/heat-transfer surface and may generate so-called scale, which must be removed at regular intervals with interruption of crystallizing operation since it deteriorates the efficiency or heat-transfer.

Moreover, since the crystals are fine and have a high water content, various problems arise during handling. For example, in the drying step, there results a product with undesirably high impurity because of the amount of mother liquor containing impurities attached to the APM crystals. The dry product also contains many fine particles or may heavily scatter in the form of fine powders.

In order to avoid such problems, the above-mentioned EP-B-91.787 proposes a crystallization method in which an aqueous APM solution is cooled through conductive heat transfer to form a pseudo solid phase without forced flow of mechanical stirring or the like, followed by further cooling of the system, where required.

By the above method, there can be obtained APM crystals having improved filtering and dewatering properties. However, the method is poor in efficiency since the cooling is effected through conductive heat transfer with no forced flow and continued even after the formation of the pseudo solid phase. Because of this, it is required to use a plurality of small crystallizers, to perform cooling over a long period of time, or to use a special crystallizer as shown in the above-mentioned patent. In this method, the temperature of places nearer to the cooling surface is lower and the temperature of places farther from the cooling surface is higher. Accordingly, the solution as a whole is heterogeneous, and its degree of supersaturation(concentration of APM in the solution minus solubility of APM) also is unevenly distributed over the contents of the crystallizer. Because of this, APM crystals are obtained which are uneven in particle size and have a wide particle size distribution.

### OBJECT OF THE INVENTION

It is an object of this invention to provide a method for crystallizing APM by cooling to obtain big APM crystals which exhibit good filtering and drying properties and hence are advantageous in production, quality and handling.

### MEANS TAKEN TO SOLVE THE PROBLEM

In view of the above, the inventors have conducted intensive investigations to improve the properties of APM crystals and, as a result, have found a surprising fact that big APM crystals having good filtering properties can be obtained by crushing crystals of APM and using the crushed crystals as seed crystals for growing crystals of APM. This invention has been completed based on the above finding.

Accordingly, this invention provides a method for crystallizing α-L-aspartyl-L-phenylalanine methyl ester which comprises crushing crystals of α-L-aspartyl-L-phenylalanine methyl ester and using the crushed crystals as seed crystals for growing crystals of α-L-aspartyl-L-phenylalanine methyl ester.

This invention will further be explained hereinbelow.

The inventors have conducted studies on the crushing of APM crystals and have found quite interestingly that APM crystals, when subjected to crushing, are broken in a specific direction. To be more specific, APM crystals are broken almost selectively with respect to their length, and hence the width of APM crystals remains almost unchanged and the length of APM crystals becomes uniformly shorter. This is a characteristic feature unique to APM crystals. This phenomenon is seen only when the crystals are subjected to crushing, not when they are subjected to simple stirring.

When the thus crushed APM crystals are used as seed crystals, the generation of new nuclei(secondary nuclei) is suppressed, and the particles of crushed crystals grow into big crystals within a shorter period of time.

Thus, the gist of this invention is to crush APM crystals and to use the crushed APM crystals as seed crystals.

In this inventon, any kind of APM crystals can be used, irrespective of the method employed for their production. The crystallization can be performed in any solvent, including water, organic solvents and water-containing organic solvents. It is however preferred, with regard to crystal growth, operation and handling, to precipitate APM crystals from an aqueous solution. In such case, a concentration of APM of 2% to 6% by weight and a pH of 3 to 6 are preferred. The APM crystals to be crushed can be precipitated either from an APM solution by evaporation of solvent, or by indirect cooling of an APM solution to utilize the difference in solubility. The precipitation can be performed by either a batch method, a semicontinuous method or a continuous method. It is possible to use as seed crystals part of a product of APM crystals which per se was produced by growing seeds of crushed APM crystals. This is advantageous in that nothing particular is required for the production of APM crystals to be used as seeds. It has also been found that big APM crystals having good filtering properties are obtained in an effective manner when APM crystals are subjected to crushing at the time when part of APM contained in an APM solution has been precipitated, and the resulting mixture is subjeced to cooling, thereby using the crushed particles of APM crystals as seed crystals for growing APM crystals. Thus, in the scope of this invention also a method for crystallizing APM is included, which comprises cooling an APM solution to partial crystallization thereof, subjecting the crystals of APM to crushing at the time when 5 to 50% by weight (based on the expected total weight of APM crystals to be precipitated) of APM has been precipitated, and then subjecting the resulting mixture to cooling, thereby using the crushed APM crystals as seed crystals for further growing of APM crystals.

The APM solution can be cooled by either direct or indirect cooling. Direct cooling can be effected, e.g. by using ice, as is described in a copending application filed by the present inventors(Japanese Patent Application No.203,703/91 ). Indirect cooling can be effected by a cooling jacket covering the crystallizer, by cooling elements positioned in the crystallizer or by a heat exchanger of an external circulation type. In these cooling devices, there can be used either water or brine. It is preferred to perform the cooling by using ice. The use of ice makes it possible to produce crystals of APM of especially good properties.

If APM crystals are present in an APM solution before it is subjected to cooling by a batch method, only an insufficient growth of seed crystals will be observed. Therefore, before cooling, the APM solution should be maintained at a temperature at which no APM crystals precipitate. Such a temperature can be readily calculated from the solubility curve of APM. In the case of an aqueous 3.5% APM solution, for example, its temperature is to be maintained before cooling at 57°C or above. Since APM solutions have a tendency to give a relatively large supersaturation, APM crystals precipitate at a temperature a lower than calculated from the solubility curve.

There are no particular restrictions on the rate of cooling. When the cooling is performed at a rate 2°C/hr or more, cooling can be completed within a short period of time. In addition, the decomposition of APM can be minimized, and crystals can be grown at a high rate since the degree of supersaturation can be high. A cooling rate of 5°C/hr or above is even more preferred.

In cases where a batch method is employed for the precipitation, the APM solution(or slurry) may, or may not, be subjected to forced flow caused by a stirrer, a circulation pump, or the like. When forced flow is applied, a smaller cooling device can be used since an improved cooling efficiency is attained. In this case, however, smaller APM crystals are obtained. On the other hand, when forced flow is not applied, a larger cooling device is required due to lower cooling efficiency. In this case, again relatively big APM crystals are obtained although the size of resulting crystals is a little nonuniform. In the case of direct cooling using ice, good cooling efficiency can be attained even without application of forced flow, and big APM crystals can be obtained. Because of this, the direct cooling method is preferably practiced without forced flow.

On the other hand, in a continuous crystallization method, forced flow in preferably utilized in order to maintain the uniformity of the APM solution or slurry.

When APM crystals are precipitated from an aqueous APM solution by a batch method, scaling is usually observed at the the time when the quantity of precipitated APM crystals exceeds 50% by weight of the quantity of APM to be precipitated. However, when APM is precipitated in accordance with the method of this invention, no scaling will be resulted even when the quantity of APM crystals exceeds 50% by weight of the quantity of APM to be precipitated. This is one of the advantages of this invention.

APM crystals to be used as seed crystals may be prepared independently by precipitating APM from an APM solution by e.g. cooling. Alternatively, an APM solution may be cooled until part of APM(5 to 50% by weight, based on the expected total weight of APM crystals to be precipitated) has been precipitated, and the resulting slurry may be subjected to crushing. The latter is more effective in efficiency since it does not require the filtration of seed crystals.

In ordinary precipitation of APM using a batch method, scaling of APM occurs quite easily, in particular, when the precipitation is performed with indirect cooling under forced flow conditions. However, in the method of this invention, the problem of scaling hardly occurs since the generation of scale can be avoided effectively by the use of seed crystals. This also applies to the method in which the seed crystals are produced in situ by crushing the APM crystals at the time when 5 to 50% by weight of APM crystals has been precipitated. The time when APM crystals are to be crushed can be determined e.g. by sampling the slurry and measuring the quantity of non-precipitated APM contained therein. If the crushing is effected before 5% by weight of APM has been precipitated only an insufficient quantity of seed crystals will be obtained, and the APM crystals will grow only insufficiently in the following step. In addition, a large number of fine crystals may be generated, and scaling may occur. On the other hand, when the crushing is effected after 50% by weight of APM has been precipitated, only an insufficient growth of the seed crystals will take place in the following step. Moreover, undesirable scaling may occur in the course of crushing.

The precipitation of APM is terminated at the time when APM crystals have been grown sufficiently and the percentage of APM precipitated has reached a sufficiently high level. The percentage of APM precipitated can be estimated from the temperature of the APM solution since the solubility of APM is defined by the temperature of the solution. Accordingly, the time at which the precipitation is to be terminated is defined by the temperature. Usually, the precipitation is terminated when the solution has been cooled to 0 to 20°C, preferably 5 to 20°C, more preferably 5 to 10°C, since any further cooling requires an excessively large cooling energy resulting only in a small increase in yield. In this manner, a slurry of well grown APM crystals can be obtained by a batch method.

APM crystals to be used as seed crystals after having been crushed can have any shape, including needles, rod-like bars and columns. The use of crystals of a shape of rod-like bars or columns is preferred since such crystals make it possible to obtain bigger APM crystals in an effective manner. Although such crystals can be obtained by various methods as stated hereinbefore, the method which utilizes direct ice cooling with no forced flow is relatively efficient and hence preferable.

The crushing of APM crystals is performed preferably by physical means. As examples of usable physical means, mention may be made of mills based on impact or friction, cutting mixers, homogenizers that utilize shearing, and ultrasonic pulverizers utilizing the impact of ultrasonic waves. Of these means, ultrasonic pulverizers, mixers and homogenizers are preferred since, by such means, APM crystals are crushed in a highly selective manner in the direction of their width.

APM crystals thus can be crushed by crushing means positioned in the crystallizer. Alternatively, the slurry of APM crystals can be externally circulated and crushed by crushing means positioned in the circulation flow. Appropriate crushing means can be selected, by taking into consideration easiness of handling, cost, efficiency, and so on. In general, the former means are suited for small crystallizers, and the latter means are suited for large crystallizers. A portable crusher or pulverizer can also be used for a small crystallizer.

It is not possible to specify general conditions for the crushing since crushing capability varies depending on the means used. The moment for terminating the crushing can be readily determined by observing the state of crushing with a microscope. At the begining of the crushing, APM crystals are broken almost uniformly with respect to their length(i.e. in the direction of their width). With the lapse or time, the APM crystals are broken into shorter particles. With further progress of crushing, the short particles of APM crystals are broken in the direction of their length(based on the original crystals). It is most preferred to terminate the crushing just before crushed pieces of APM crystals start to be broken in the direction of their length(based on the original crystals). If termination of crushing is too early, an unnecessarily large quantity of seed crystals will be required, whereas if it is too late too many undesirably small crystals will be obtained.

In the case where a mixer or homogenizer is used, the degree of crushing can be adjusted, e.g. by changing the shape of blades, the number of rotations and/or the duration of rotation. In the case where an ultrasonic pulverizer is used, it can be adjusted, e.g. by changing the frequency, the size of vibrator, the distance between the vibrator and the APM crystals, energy output and/or duration of crushing. When the crushing is performed at high supersaturation, the quantity of crystals increases even during the course of crushing. However, such increase does not cause problems of scaling since most of the increase is consumed by the growth of crystals.

The crushed APM crystals prepared in the above way are then used as seed crystals for growing APM crystals. The crushed seed crystals grow quite well, and big APM crystals are obtained within a short period of time.

It is difficult to specify exactly the quantity of seed crystals to be used since it varies depending on their shape and size, and the conditions for growing the seeds. However, good results are obtained by using seed crystals in an amount of 5 to 50% by weight, in particular, 10 to 35% by weight(based on the expected total weight of APM crystals to be precipitated ). The expected total weight of APM crystals to be precipitated may be estimated from the relationship between the solubility of APM and the temperature at which the precipitation is terminated. When the quantity is less than 5% by weight, undesirable generation of now crystal nuclei may result although the load for producing seed crystals can be reduced and bigger crystals can be obtained with an increased efficiency.

On the other hand, when the quantity of seed crystals is larger than 50% by weight, the load for producing seed crystals is greater and it is difficult to obtain big APM crystals although no new nuclei are generated.

It is therefore preferred to use seed crystals in an amount where the seed crystals can be grown without substantial generation of new nuclei; thus, although the amount of seed crystals can be small at the same time big APM crystals having good filtering properties can be obtained.

In the case where separately prepared seed crystals are added to the crystallization system, the seed crystals may be added either in the form of a slurry or as a powder. It is however preferred to add seed crystals in the form of a slurry. The seed crystals can be added continuously or intermittently, or at once. It is preferred to add seed crystals to an APM solution saturated or supersaturated with APM. When seed crystals are added to an unsaturated APM solution, part of the seed crystals added will be dissolved. In certain cases, however, such dissolution may contribute to the elimination of undesirably fine seed crystals. When seed crystals are grown by a continuous method, the precipitation system is always in a supersaturated condition, and hence the problem of dissolution will not arise.

The temperature of the crystallizer in which the seed crystals are grown is preferably in the range of 0 to 50°C, more preferably 3 to 20°C. When the temperature is higher than 50°C, impurities may be formed by the decomposition of APM, whereas when the temperature is lower than 0°C, the load for the cooling becomes unnecessarily large, and there may be resulted an insufficient growth of the seed crystals.

The seed crystals may be grown by either a batch method, a semi-continuous method, or a continuous method.

In the case where APM crystals are produced through cooling by a batch method, the first half of the process may be used for the preparation of seed crystals, and the second half of the process may be used for the growing of seed crystals. To be more specific, crystals of APM are for example precipitated by cooling an APM solution by a batch method. At the time when part of the APM crystals has been precipitated, preferably up to an amount of 5 to 50% by weight, more preferably 10 to 35% by weight(based on the expected total weight of APM crystals to be precipitated), they are subjected to crushing, to form seed crystals. Thereafter, the precipitation system is subjected to further cooling to allow the seed crystals to grow. The thus obtained slurry of grown APM crystals is then subjected to filtration by either a batch method or a continuous method. In any case, a high dewatering rate is attained within a short period of time. For the filtering any filtering apparatuses conventionally employed in the industry, including centrifugal separators, filter presses, belt filters, drum filters, and the like can be used. If desired, the filtered product may be washed with water or an APM solution. The washing is easy. The quality of APM crystals is improved by washing since the mother liquid attached to the crystals is removed by this operation. The wet cake obtained by the filtering can be dried either as it is or after having been granulated. For the drying, any type of drier can be used, such as a flash drier, a fluidized-bed drier or a rotary drier.

In this invention, APM crystals which are big in size and hence exhibit good filtering and drying properties are produced efficiently. Although the reason for this is not fully clear, it is presumed to be as follows:

Attempts have already been made to use APM crystals as seed crystals, however without crushing, as is described EP-B-91.787; Chemistry and Industry, Feb. 16, 1987, p.127-128; and J. Chem. Tech. Biotechnol., 1988, 43, p.71-82. However, none of these attempts yielded good results. This is because APM crystals used as seed crystals used in the above attempts must have been in the form of needles, and hence of each needle the area of growth point(its 'point area') is small. Because of this, when an APM solution containing such seed crystals is cooled, the generation of secondary nuclei preferentially takes place, rather than further growth of the seed crystals. Thus, undesirable precipitation of a large number of fine crystals results, with no significant growth of the seed crystals. This is presumably the reason why good results could not be attained in the above attempts.

On the contrary, in this invention APM crystals are subjected to crushing, and the crushed crystals are used as seed crystals. As is described hereinbefore, APM crystals, when subjected to crushing, are selectively broken with respect to their length to form shorter crystals. Thus the ratio of width to length increases. When the seed crystals are grown, the generation of secondary nuclei is suppressed owing to the large number of growth points which appear at new faces due to breaking. Although the seed crystals grow preferentially in the direction of their length, the resulting APM crystals become relatively bigger and have a greater width/length ratio, in comparison with those prepared by using uncrushed seed crystals. This is presumably the reason why APM crystals having good filtering and drying properties are obtained efficiently.

### EXAMPLES

This invention will hereinafter be explained by means of examples and comparative examples. It should however be noted that this invention is by no means limited to these examples.

In the following examples and comparative examples, the filtering rate of APM crystals was measured in the following manner.

By using a suction filter(leaf tester) fitted with a polypropylene cloth filter having a permeability of air of 5ml/cm²·sec(12mmH₂O), 500ml of slurry containing precipitated APM crystals was filtered at -400mmHg, whereby the slurry was poured carefully onto the filter so that the filtering could be performed continuously with no drying up of the slurry on the filter cloth throughout the operation. The filtering rate was calculated from the period of time lapsed from the start to the completion of the filtering(the filtering was considered complete when the solution of the slurry no more remained on the filter cloth).

### Example 1

Into a 2 liter glass flask 1kg of aqueous 3.5 wt% APM solution of 60°C(pH=4.5) was charged, and then 500g of ice cubes(ca. 30mmX30mmX25mm) was added thereto at once. Sixty(60) minutes later, a blade-type stirrer was mounted, and the contents of the flask were stirred to give a uniform slurry when the ice had melted almost completely and the precipitation of APM crystals had almost ceased by the time. The thus obtained APM crystals were in the form of rod-like bars or columns having a width of 5 to 35µm and a length of 100µm or above.

The slurry was then charged into a home-use mixer(MX-B30 G manufactured by Toshiba Corp.) and crushed for 1 minute to give seed crystals. After the crushing, the APM crystals had a width of 3 to 35µm and length of 30 to 150µm, and the concentration of the APM crystals was 1.4% by weight.

Into a glass flask(internal volume: 2.5 liters) equipped with an external cooling jacket and stirrer 1.56kg of aqueous 3.5 wt% APM solution of 60°C(pH=4.5) was charged. While being stirred at 300 r.p.m., the solution was cooled at a rate of 15°C per hour. When the solution had been cooled to 52°C(by the point of time, the solution had become supersaturated with APM, and APM crystals had not yet been precipitated), 780g of the 2.4% slurry of the crushed APM crystals prepared above was added thereto at once, and cooling was continued to 10°C.

The crushed seed crystals grew well, and no substantial scale was generated. The resulting APM crystals had a width of 5 to 50µm and a length of 100µm or above, and the concentration of the slurry was 2.4% by weight. The slurry gave an excellent filtering rate as high as 340 liters/m²·min.

### Comparative Example 1

APM crystals were produced in the same manner as in Example 1, only except that no crushed seed crystals were added and 2 liters of aqueous APM solution were used.

The thus obtained APM crystals were in the form of fine needles having a width of 10µm or less and a length of 30 to 100µm, and a large quantity of scale was generated. The scale was stripped off and admixed with the slurry to give a combined uniform slurry. The filtering rate of the resulting slurry was as poor as 88 liters/m²·min.

### Example 2

Into a 2 liter beaker 1.5 liters of aqueous 3.5 wt% APM solution of 60°C(pH=4.5) was charged. The beaker was sealed with Saran Wrap(trade name) and allowed to stand in a refrigerator of Ca. 10°C.

On the following day, the contents of the beaker, which were in a "sherbet-like" state, were disintegrated with a blade-type stirrer to give a uniform slurry. APM crystals contained in the slurry were in the form of rod-like bars or columns having a width of 5 to 40µm and a length of 100µm or above.

The slurry was then subjected to the crushing in the same manner as in Example 1 to give seed crystals. After the crushing, the APM crystals had a width of 3 to 40µm and a length of 30 to 150µm, and the concentration of the APM crystals was 2.65% by weight.

Then, 420g of slurry of the seed crystals was treated in the same manner as in Example 1, except that 1.58kg of aqueous 3.5wt% APM solution(pH=4.5) was used.

The seed crystals grew well, and no substantial scale was generated. The resulting APM crystals had a width of 5 to 50µm and a length or 100µm or above, and the APM crystals concentration of the slurry was 2.65% by weight. The slurry gave an excellent filtering rate as high as 370 liters/m²·min.

### Example 3

Into a cylindrical vessel(inner diameter 32mm; height 400mm) equipped with a cooling jacket 300ml of aqueous 3.5 wt% APM solution of 60°C(pH=4.5) was charged, and cold water of 5°C was circulated through the cooling jacket, during which the vessel was set aside.

After 1 hour of cooling, the contents of the vessel, which were in the state of a sherbet-like slurry, were taken out from the bottom of the container, and disintegrated with a blade-type stirrer. The temperature of the resulting slurry was 8°C, and APM crystals contained in the slurry were in the form of rod-like bars or columns having a width of 5 to 40µm and a length of 100µm or above. The slurry was then subjected to crushing in the same manner as in Example 1 to give seed crystals. After the crushing, the APM crystals had a width of 3 to 40µm and length of 30 to 150µm, and the concentration of the APM crystals was 2.7% by weight. These procedures were repeated three times.

Then, APM crystals were grown in the same manner as in Example 1, by using 410g of slurry of the seed crystals and 1.59kg of aqueous 3.5 wt% APM solution(pH=4.5).

The seed crystals grow well, and no substantial scale was generated. The resulting APM crystals had a width of 5 to 50µm and a length of 100µm or above, and the APM crystals concentration of the slurry was 2.7% by weight. The slurry gave an excellent filtering rate as high as 360 liters/m²·min.

### Example 4

The slurry obtained in Comparative Example 1 was subjected to crushing in the same manner as in Example 1 to give seed crystals. After the crushing, the APM crystals had a width of 10µm or less and a length of 20 to 50µm.

Then, APM crystals were grown in the same manner as in Example 1, by using 200g of slurry of the seed crystals and 1.8 kg of aqueous 3.5 wt% APM solution(pH=4.5).

The seed crystals grew well, and no substantial scale was generated. The resulting APM crystals had a width of 15µm or less or less and a length of 50 to 150µm, and the APM crystals concentration of the slurry was 2.7% by weight. The filtering rate of the slurry was 135 liters/m²·min.

### Comparative Example 2

APM crystals were produced in the same manner as in Example 4, except that 200g of slurry of APM crystals prepared as in Example 1 was used as seed crystals without crushing.

The growth of the seed crystals was poor, and APM crystals so obtained were in the form of fine crystals having a width of 10µm or less and a length of 30 to 110µm, and a substantial quantity of scale was generated. The scale was stripped off and admixed with the slurry to give a combined uniform slurry. The filtering rate of the resulting slurry was as poor as 90 liters/m²·min.

### Comparative Example 3

The procedure of Example 1 was repeated, except that the seed APM crystals were not crushed.

As a result, there was obtained a slurry containing APM crystals having a width of 35µm or less and a length of 30µm or above. In addition, a large number of fine crystals of APM was present in the slurry, and a substantial quantity of scale was also generated. The scale was stripped off and admixed with the slurry to give a combined uni orm slurry. The filtering rate of the resulting slurry was 145 liters/m²·min.

### Comparative Example 4

The procedure of Example 2 was repeated, except that the APM seed crystals were not crushed.

Like in Comparative Example 3, there was obtained a slurry containing APM crystals having a width of 35µm or less and a length of 30µm or above. In addition, a large number of fine crystals of APM were present in the slurry, and a substantial quantity of scale was also generated. The scale was stripped off and admixed with the slurry to give a combined uniform slurry. The combined slurry gave a filtering rate of 150 liters/m²·min.

### Example 5

Into a beaker 500g of slurry of rod-like bar- or column- shaped APM crystals prepared as in Example 1 was charged, and the crystals were subjected to crushing for 1.5 minutes by use of an ultrasonic cleaner(Sono Cleaner CA-20, manufactured by Kaijo Denki K.K.), to give seed crystals. After the crushing, the APM crystals had a width of 3 to 35µm and a length of 20 to 150µm. The concentration of the APM crystals was 1.4% by weight. Then, APM crystals were grown in the same manner as in Example 1, by using 780g of slurry of the seed crystals.

The seed crystals grew well, and no substantial scale was generated.

The resulting APM crystals had a width of 5 to 50µm and a length of 100µm or above, and the APM crystals concentration of the slurry was 2.4% by weight. The slurry gave an excellent filtering rate as high as 360 liters/m²·min.

### Example 6

Into a glass flask(internal volume: 2.5 liters) equipped with an external cooling jacket and stirrer 1.56kg of aqueous 3.5 wt% APM solution of 60°C(pH=4.5) was charged. While being stirred at 300 r.p.m., the solution was cooled at a rate of 15°C per hour. APM crystals were precipitated at 42°C, and then the cooling of APM solution was interrupted.

APM crystals were in the form of rod-like bars or columns having a width of 5 to 15µm and a length of 100µm or above.

The total amount of the slurry was then rapidly charged into a home-use mixer(MX-B30G manufactured by Toshiba Corp.) which was warmed previously to 60°C , and the slurry was crushed for 1 minute to give seed crystals. After crushing, the APM crystals had a width of 3 to 15µm and a length of 20 to 100µm, and the concentration of the APM crystals was 0.95% by weight.

This crushed slurry was then charged again into the 2.5 liters flask mentioned above, and while being stirred at 300 r.p.m., the solution was cooled to 15°C at a rate of 15°C per hour. The crushed seed crystals grew well, and no substantial scale was generated. The resulting APM crystals had a width of 5 to 20µm and a length of 5 to 200µm, and the APM crystals concentration of the slurry was 2.7% by weight. The slurry gave an excellent filtering rate as high as 150 liters/m²·min.

### Example 7

The crushing of APM crystals was repeated in the same manner as in Example 6, except that the crushing was carried out at 40°C.

Before and after crushing the shapes of APM crystals were similar to those in Example 6, and the concentration of the crushed seed APM crystals was 1.2%. The crushed seed APM crystals grew well and no substantial scale was generated. The resulting APM crystals had a width of 5 to 20µm and a length of 50 to 200µm, and the concentration of slurry was 2.7% by weight. The slurry gave an excellent filtering rate as high as 140 liters/m²·min.

### Comparative example 5

The procedure was repeated in the same manner as in Example 6, except that the seed APM crystals were not crushed.

The resulting APM crystals had a width of 10µm or less and a length of 30 to 100µm, and the shape of fine crystals. In addition, a substantial quantity of scale was generated. The scale was stripped off and admixed with the slurry to give a combined uniform slurry. The filtering rate of the resulting slurry was 88 liters/m²·min.

### Example 8

Into a glass flask(internal volume: 2.5 liters) equipped with an external cooling jacket and stirrer 1.56kg of aqueous 3.5 wt% APM solution of 60°C(pH=4.5) was charged, and 300g of ice cubes(30mmX30mmX25mm) were added thereto at once, and the system was left without forced flow. The ice almost had melted after 1 hour, and APM crystals having a width of 5 to 35µm and a length of 30 to 150µm had precipitated in the form of rod-like bars and columns in the upper part of the flask; the slurry then was disintegrated with the (blade-type) stirrer to give a uniform slurry. The temperature of the slurry was 37.2°C.

The homomixer(manufactured by Tokushu Kika Industry Ltd.) was inserted in the flask, and the slurry was crushed for 1 minute with stirring at 10,000 r.p.m. to obtain APM seed crystals. The crushed APM seed crystals had a width of 3 to 35 µm and length of 30 to 150µm, and the concentration of the slurry was 0.57% by weight.

The stirrer was reinstalled on the flask, and the slurry was cooled to 10°C at a rate of 15°C per hour with stirring at 300 r.p.m.. The crushed APM seed crystals grew well, and no substantial scale was generated.

The resulting APM crystals had a width of 5 to 50µm and length of 100µm or above, and the concentration of the slurry was 2.1% by weight. The slurry gave an excellent filtering rate as high as 345 liters/m²·min.

### Comparative example 6

The procedure was repeated in the same manner as in Example 8, except that the APM crystals were not crushed.

The resulting APM crystals had a width of 35µm or less and a length of 30µm or above, and a substantial quantity of scale was generated. The scale was stripped off and admixed with the slurry to give a combined uniform slurry. The filtering rate of the resulting slurry was 150 liters/m²·min.

### Example 9

Into a glass flask(internal volume: 2.5 liters) equipped with an external cooling jacket and stirrer 1.56kg of aqueous 3.5 wt% APM solution of 60°C(pH=4.5) was charged, and water of 10 ° C was circulated through the external cooling jacket without stirring in the flask. APM crystals were precipitated near the cooling conductive surfaces and the APM crystals were precipitated only slowly at points far from the surfaces. The scale which had formed on the conductive surfaces was stripped off after 0.5 hour from the time when the APM began to precipitate, and this scale was admixed with the slurry to give a combined uniform slurry. The temperature was 39.5°C in the flask, and the APM crystals having a width of 5 to 50µm, a length of 100µm or above, and shapes of rod-like bars or columns were obtained.

An ultrasonic vibrator(Type:US300 manufactured by Nihon Seiki Manufacturing Co.,) was inserted in the flask, and crushing of APM crystals was carried out for 1 minute while circulating cooled water through the external cooling jacket. The APM crystals obtained by crushing had a width of 3 to 40µm and a length of 30 to 50µm, and the APM crystal concentration was 0.95% by weight.

The slurry of APM crystals was then cooled to 10°C at a rate of 15°C per hour with stirring at 300 r.p.m.. The crushed APM seed crystals grew well and no substantial scale was generated. The resulting APM crystals had a width of 5 to 50µm and a length of 100µm or above, and the concentration of slurry was 2.7% by weight. The slurry gave an excellent filtering rate as high as 360 liters/m²·min.

### Comparative example 7

The procedure was repeated in the same manner as in Example 9, except that the seed APM crystals were not crushed.

The resulting APM crystals had a width of 40µm or less and a length of 30µm or above, and a substantial quantity of scale was generated. The scale was stripped off and admixed with the slurry to give a combined uniform slurry. The filtering rate of the resulting slurry was 150 liters/m²·min.

### ADVANTAGEOUS EFFECTS OF THE INVENTION

In this invention, APM crystals are crushed, and crushed crystals are used as seed as seed crystals for growing APM crystals. Because of this, the generation of fine secondary crystals can be suppressed and big APM crystals can be obtained. Thus the filtering and drying properties of the crystals are improved. Such crystals are advantageous with regard to production, quality and handling. In addition, they can be precipitated in quite an efficient manner by using only a small quantity of seed crystals.

Other advantages of this invention are as follows.
(1) Since the quantity of seed crystals to be used can be small, they can be produced by using compact equipment. Part of crystals grown can be used as seed crystals and, in this case,a further improved efficiency is attained;
(2) Seed crystals can be grown in one step, and the operation can be performed quite easily even under forced flow, by using a compact, easily operational apparatus. In addition, an industrial large-scale production can be carried out with less consumption of energy;
(3) It is also possible to prepare, crush and grow seed crystals in one single crystallizer. In this case, too, APM crystals can be produced on an industrial scale with a high energy efficiency;
(4) APM crystals prepared by growing crushed seed crystals are big in size. Such APM crystals can be filtered and washed easily. Because of this, a wet APM cake having low water content and hence contaminated with only a small quantity of impurities can be obtained within a short period time;
(5) APM crystals can be grown without suffering from the problem of scaling. Scaling is suppressed even when APM crystals are grown under forced flow;
(6) Since the quantity of water attached to a wet APM cake can be reduced, the cake can be easily dried at low temperature with less drying energy and within a shorter period of time. Futhermore, a high-quality APM product can be obtained since it is free from deterioration in quality upon drying of the cake. In addition, in the drying step, dust of fine APM crystals is generated only in a small quantity, which can be advantageous with regard to operation; and
(7) The dried final product gives only a small quantity of dust of fine APM crystals, which can be highly advantageous with regard to its handling.

As is described hereinabove, this invention provides a method for crystallizing APM crystals which is advantageous with regard to operation, cost and quality.

## Claims

1. A method for crystallizing alpha-L-aspartyl-L-phenylalanine methyl ester by cooling, characterized in that crystals of alpha-L-aspartyl-L-phenylalanine methyl ester are crushed and that the crushed crystals are used as seed crystals for growing crystals of alpha-L-aspartyl-L-phenylalanine methyl ester.

2. A method according to claim 1 wherein the seed crystals are obtained by crushing the slurry resulting from a first precipitation of 5 to 50% of total weight of crystals of alpha-L-aspartyl-L-phenylalanine methyl ester to be precipitated in total from the solution.

3. A method according to claim 1 or claim 2 wherein the concentration of alpha-L-aspartyl-L-phenylalanine methyl ester is in the range of 2 to 6% by weight.

4. A method according to any of claims 1 to 3 wherein the pH of the solution of alpha-L-aspartyl-L-phenylalanine methyl ester is in the range of 3 to 6.

5. A method according to any of claims 1 to 4 wherein the cooling rate is 2°C/hr or more.

6. A method according to any of claims 1 to 5 wherein a solution of alpha-L-aspartyl-L-phenylalanine methyl ester is cooled to a temperature in the range of 0 to 20°C.

7. A method according to any of claims 1 to 6 wherein cooling of said solution takes place by direct cooling using ice.

## Patentansprüche

1. Verfahren zur Kristallisation von α-L-Aspartyl-L-phenylalaninmethylester durch Kühlen, dadurch gekennzeichnet, daß Kristalle von α-L-Aspartyl-L-phenylalaninmethylester zerkleinert werden, und daß die zerkleinerten Kristalle als Impfkristalle zum Züchten von Kristallen von α-L-Aspartyl-L-phenylalaninmethylester verwendet werden.

2. Verfahren nach Anspruch 1, bei welchem die Impfkristalle erhalten werden durch das Zerkleinern der Aufschlämmung, die aus einer ersten Ausfällung von 5 bis 50 % der Gesamtmasse der Kristalle von α-L-Aspartyl-L-phenylalaninmethylester resultiert, um insgesamt aus der Lösung ausgefällt zu werden.

3. Verfahren nach Anspruch 1 oder 2, bei welchem die Konzentration von α-L-Aspartyl-L-phenylalaninmethylester im Bereich von 2 bis 6 Masse-% liegt.

4. Verfahren nach einem der Ansprüche 1 bis 3, bei welchem der pH der Lösung von α-L-Aspartyl-L-phenylalaninmethylester im Bereich von 3 bis 6 liegt.

5. Verfahren nach einem der Ansprüche 1 bis 4, bei welchem die Kühlrate 2°C/h oder mehr beträgt.

6. Verfahren nach einem der Ansprüche 1 bis 5, bei welchem eine Lösung von α-L-Aspartyl-L-phenylalaninmethylester auf eine Temperatur im Bereich von 0 bis 20°C abgekühlt wird.

7. Verfahren nach einem der Ansprüche 1 bis 6, bei welchem das Kühlen der Lösung durch direkte Kühlung unter Verwendung von Eis stattfindet.

## Revendications

1. Procédé de cristallisation de l'ester méthylique d'α-L-aspartyl-L-phénylalanine par refroidissement, caractérisé en ce que les cristaux d'ester méthylique d'α-L-aspartyl-L-phénylalanine sont broyés et en ce que les cristaux broyés sont utilisés comme germes cristallins pour la croissance de cristaux d'ester méthylique d'α-L-aspartyl-L-phénylalanine.

2. Procédé selon la revendication 1, dans lequel les germes cristallins sont obtenus par broyage de la suspension résultant d'une première précipitation de 5 à 50% du poids total des cristaux d'ester méthylique d'α-L-aspartyl-L-phénylalanine à précipiter au total dans la solution.

3. Procédé selon la revendication 1 ou la revendication 2, dans lequel la concentration en ester méthylique d'α-L-aspartyl-L-phénylalanine se situe dans la gamme de 2 à 6% en poids.

4. Procédé selon l'une quelconque des revendications 1 à 3, dans lequel le pH de la solution d'ester méthylique d'α-L-aspartyl-L-phénylalanine se situe dans le domaine de 3 à 6.

5. Procédé selon l'une quelconque des revendications 1 à 4, dans lequel la vitesse de refroidissement est de 2°C/h ou plus.

6. Procédé selon l'une quelconque des revendications 1 à 5, dans lequel une solution d'ester méthylique d'α-L-aspartyl-L-phénylalanine est refroidie à une température dans la gamme de 0 à 20°C.

7. Procédé selon l'une quelconque des revendications 1 à 6, dans lequel le refroidissement de ladite solution à lieu par refroidissement direct avec de la glace.
